# EUROPEAN PATENT APPLICATION

(11) **EP 2 767 303 A1**
(43) Date of publication of application: **20.08.2014**
(21) Application number: 14152293.8
(22) Date of filing: 23.01.2014
(51) Int. Cl.: A61M 25/00

(54) **Catheter**

(30) Priority: 15.02.2013 JP 2013028088
(71) Applicant: ASAHI INTECC CO., LTD., Nagoya-shi, Aichi 463-0024 (JP)
(72) Inventor: Miyata, Naohiko, Nagoya-shi, Aichi 463-0024 (JP); Oshima, Fumiyoshi, Nagoya-shi, Aichi 463-0024 (JP)
(74) Representative: Winter, Brandl, Fürniss, Hübner, Röss, Kaiser, Polte - Partnerschaft mbB

(57) **Abstract**

In a catheter (1), since the thickness of an intermediate layer (29) is constant from a body (10a) to a distal end portion (10b), the adhesive strength (adhesiveness) between the intermediate layer (29) and an inner layer (24) is uniform from the body (10a) to the distal end portion (10b) of a catheter shaft (10). Therefore, when the catheter (1) is curved, the movement of a braid (26) caused by the curving of the catheter (1) can be uniformly suppressed from the body (10a) to the distal end portion (10b) that is adjacent thereto. Even if, when the catheter (1) is curved, a tensile stress (30a) towards a proximal end acts upon a first outer layer (28a) at the body (10a) and a tensile stress (30b) towards a distal end acts upon a second outer layer (28b) at the distal end portion (10b) adjacent to the body (10a), the first and second outer layers (28a, 28b) are caught by an uneven contour of the intermediate layer (29) by an anchor effect, so that it is possible to prevent peeling of the first and second outer layers (28a, 28b) from an intermediate layer (29).

## Description

### BACKGROUND OF THE INVENTION

### 1. Field of the Invention

The present invention relates to a catheter that is inserted into a blood vessel, and, particularly, to a catheter including outer layers having different rigidities.

### 2. Description of the Related Art

A catheter that is inserted into a blood vessel is a long and narrow medical device having a flexible structure. A catheter is used as an examining device that diagnoses a disease (such as identifying the location of a stenosis that requires treatment or determining the degree of development of the stenosis), or as a treating device that treats a disease (such as treating a stenosis or injecting a medicine into the stenosis).

In general, an examining catheter and a treating catheter both include an inner layer, an outer layer, and a braid. The inner layer is formed of a resin. The outer layer is formed of a resin and covers an outer periphery of the inner layer. The braid is a reinforcing member in which a metallic wire is braided between the inner layer and the outer layer. Various capabilities, such as flexibility of a distal end, thrusting-in capability, torque rotatability, kink resistance, and followability, are required of a catheter.

For example, as a catheter whose flexibility of a distal end and thrusting-in capability have been considered, there is known a catheter including an outer layer having different rigidities by forming a proximal end portion of the outer layer (body of a catheter shaft) out of a highly rigid resin and forming a distal end portion of the outer layer (distal end portion of the catheter shaft) out of a flexible resin (see, for example, US Patent No. 5254107 (PTL 1) and Japanese Patent No. 4741151 (PTL 2)).

In PTL 1, outer layers having different rigidities are formed by forming a braid at an outer periphery of an inner layer that is formed of a resin, inserting the outer layer that is formed of a flexible resin and the outer layer that is formed of a resin having a high rigidity into an outer periphery of the braid, and subjecting them to thermal welding. The outer layer that is formed of a flexible resin has a shore hardness D of 35 to 45 and the outer layer that is formed of a resin having a high rigidity has a shore hardness D of 65 to 70. In PTL 2, outer layers having different rigidities are formed by forming a braid at an outer peripheral surface of an inner layer that is formed of a resin; temporarily forming the outer layer that is formed of a resin having a high rigidity to an outer periphery of the braid; then, removing only a distal end portion of this outer layer; and replacing it with the outer layer that is formed of a flexible resin.

In this way, when outer layers having different rigidities are used, it is possible to ensure flexibility at a distal end and thrusting-in capability. However, when a catheter is inserted into a meandering blood vessel and is placed in a curved state for a long time, stress is concentrated at a boundary between the outer layers having different rigidities (that is, the boundary between the body and the distal end portion of the catheter shaft), as a result of which the outer layers may crack or may be peeled.

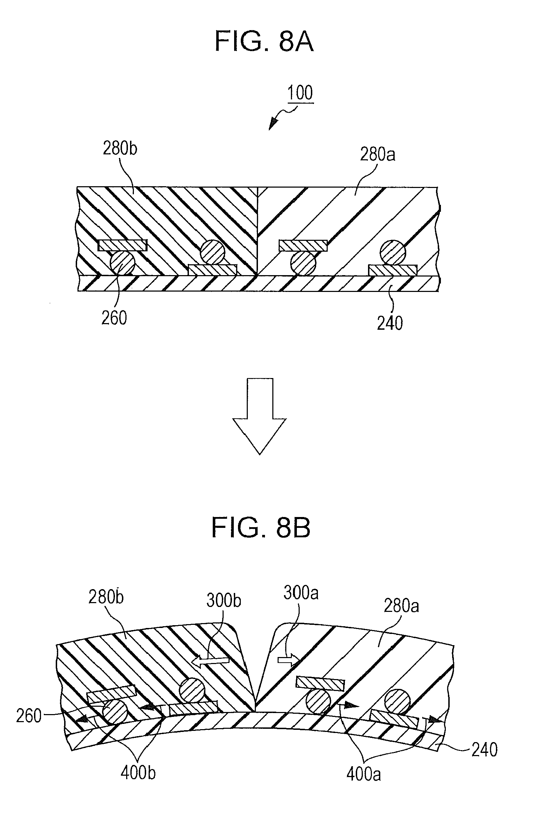

For example, as shown in Figs. 8A and 8B, when a catheter 100, in which an outer periphery of a braid 260 that is formed at an outer periphery of an inner layer 240 is covered with a first outer layer 280a (formed of a resin having a high rigidity) and a second outer layer 280b (formed of a flexible resin), is curved, the following problems occur. Since the adhesive strength (adhesiveness) between the first outer layer 280a and the inner layer 240 differs from the adhesive strength (adhesiveness) between the second outer layer 280b and the inner layer 240, the degrees of suppression of the movements of the braid 260 differ from each other. The movements of the braid 260

(that is, the increasing of the pitch between portions of the braid 260) caused by the curving of the catheter 100 is greater for the portion of the braid 260 within the second outer layer 280b that is formed of a flexible resin than for the portion of the braid 260 within the first outer layer 280a that is formed of a highly rigid resin (in other words, the amounts of movements of the braid 260 are such that 400a < 400b). Therefore, a tensile stress 300a that pulls the first outer layer 280a towards a proximal end and a tensile stress 300b that pulls the second outer layer 280b towards a distal end are not equal to each other. Stress is concentrated at a boundary between the first outer layer 280a and the second outer layer 280b having different rigidities, as a result of which cracking occurs or the second outer layer 280b is peeled off from the inner layer 240.

### SUMMARY OF THE INVENTION

Accordingly, in view of the above-described situation, it is an object of the present invention to provide a catheter that can prevent cracking or peeling of outer layers by suppressing stress that is generated at a boundary between the outer layers having different hardnesses (for example, a boundary between a body of a catheter shaft and an adjacent portion that is adjacent to the body) even if the catheter inserted in a blood vessel is placed in a curved state for a long time.

The object is achieved by a catheter in accordance with claim 1. Claims 2 to 6 refer to embodiments of the catheter.

According to the present invention, there is provided a catheter including an inner layer formed from a body to an adjacent portion that is adjacent to the body, the inner layer being formed of a resin; a braid formed at an outer periphery of the inner layer from the body to the adjacent portion; an intermediate layer with which an outer periphery of the braid is covered from the body to the adjacent portion; a first outer layer with which an outer periphery of the intermediate layer is covered at the body, the first outer layer being formed of a resin; and a second outer layer with which an outer periphery of the intermediate layer is covered at the adjacent portion and that is formed of a resin that is more flexible than the first outer layer. In the catheter, the intermediate layer has a contour including a protrusion and a recess from the body to the adjacent portion. The protrusion is formed at an upper side of the braid, and the recess is formed at a gap of the braid adjacent to the protrusion. In addition, in the catheter, a thickness of the intermediate layer at the gap is constant from the body to the adjacent portion.

In the catheter according to an aspect of the present invention, the intermediate layer has an uneven contour from the body to the adjacent portion, and the thickness of the intermediate layer at the gap of the braid is constant from the body to the adjacent portion. Therefore, the adhesive strength (adhesiveness) between the inner layer and the intermediate layer that covers the braid becomes uniform from the body to the adjacent portion; and, when the catheter is curved, the movements of the braid (that is, the increasing of the pitch between portions of the braid 260) caused by the curving of the catheter can be uniformly suppressed from the body to the adjacent portion. In addition, even if, when the catheter is curved, tensile stress towards the proximal end acts upon the first outer layer at the body and tensile stress towards the distal end acts upon the second outer layer at the adjacent portion, the first and second outer layers are caught by the uneven contour of the intermediate layer by an anchor effect, so that it is possible to prevent peeling of the first and second outer layers from the inner layer. Therefore, even if the catheter is placed in a curved state for a long time, it is possible to reduce the possibility of cracking at the boundary between the body and the adjacent portion and the possibility of peeling of the outer layers.

### BRIEF DESCRIPTION OF THE DRAWINGS

Fig. 1 is an overall view of a catheter according to an embodiment.
Fig. 2 illustrates a catheter shaft according to the embodiment. For explanatory purposes, a distal-end tip, part of an intermediate layer, and part of an outer layer are not shown in Fig. 2.
Fig. 3 is a sectional view of the catheter shaft and the distal-end tip according to the embodiment.
Figs. 4A and 4B are each an enlarged sectional view of a body and a distal end portion of the catheter shaft according to the embodiment, with Fig. 4A showing a state before the catheter shaft is curved and Fig. 4B showing a state after the catheter shaft is curved.
Figs. 5A and 5B are each an enlarged sectional view of a body and a distal end portion of a catheter shaft according to another embodiment, with Fig. 5A showing a state before the catheter shaft is curved and Fig. 5B showing a state after the catheter shaft is curved.
Fig. 6 illustrates the catheter shaft shown in Fig. 2 that is curved.
Fig. 7 illustrates a process for forming a catheter shaft according to an embodiment.
Figs. 8A and 8B are each an enlarged sectional view of a body and a distal end portion of an existing catheter shaft.

### DESCRIPTION OF THE PREFERRED EMBODIMENTS

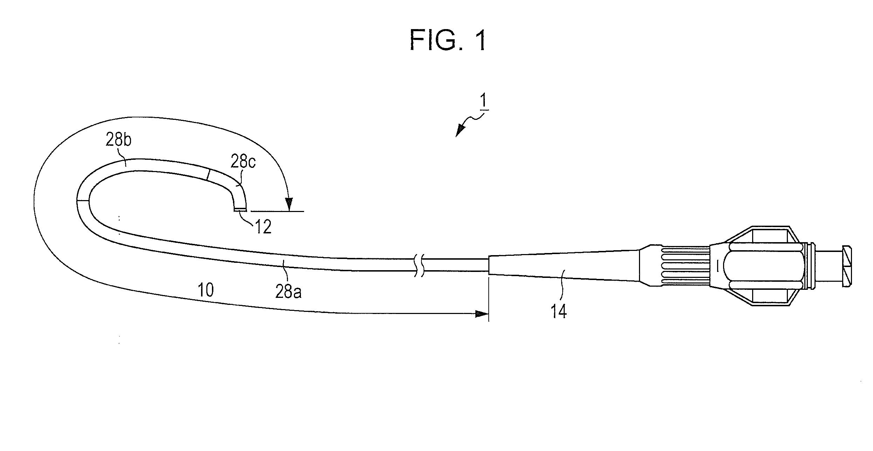

With reference to Figs. 1 to 8B, catheters 1 according to embodiments are described. In Figs. 1 to 3 and Figs. 6 and 7, the illustrated left side corresponds to a distal end side that is inserted into a human body and the illustrated right side corresponds to a proximal end side (base end side) that is operated by an operator such as a doctor.

The catheter 1 shown in Fig. 1 is a tubular medical device whose overall length is approximately 1200 mm. The catheter 1 primarily includes a flexible catheter shaft 10, a distal-end tip 12 bonded to a distal end of the catheter shaft 10, and a connector 14 secured to a proximal end portion of the catheter shaft 10.

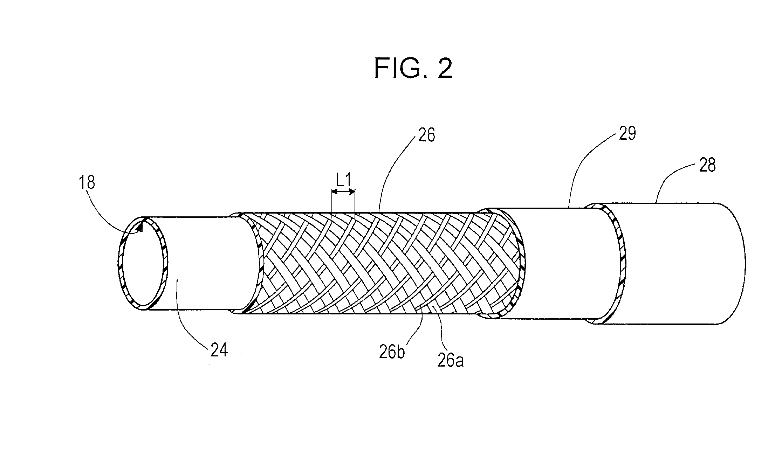

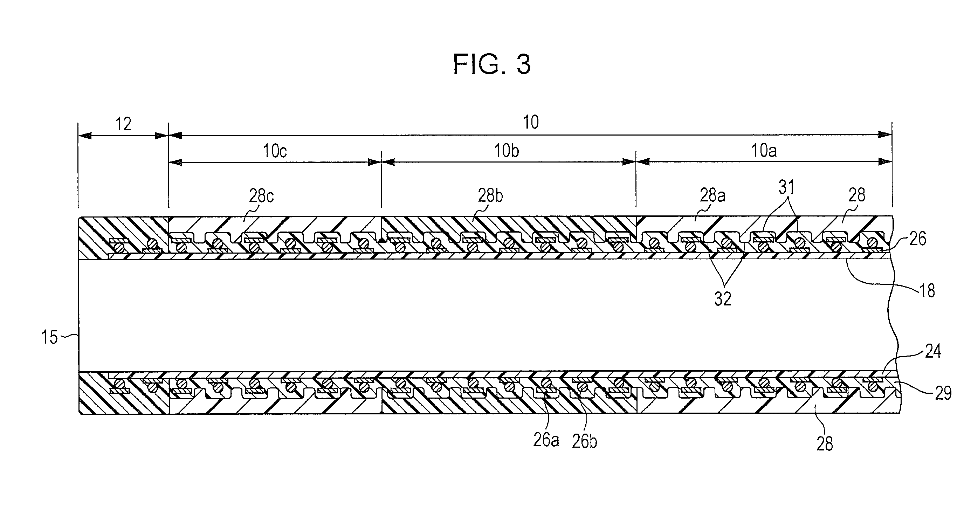

As shown in Figs. 2 and 3, the catheter shaft 10 includes, from an inner side of the catheter shaft 10 in a radial direction, an inner layer 24, a braid 26 (serving as a reinforcing member), an intermediate layer 29, and an outer layer 28.

The inner layer 24 is formed of a resin and forms a lumen in an inner portion thereof for inserting a guidewire or another catheter. Although the resin material of the inner layer 24 is not particularly limited, polytetrafluoroethylene (PTFE) is used in the embodiment.

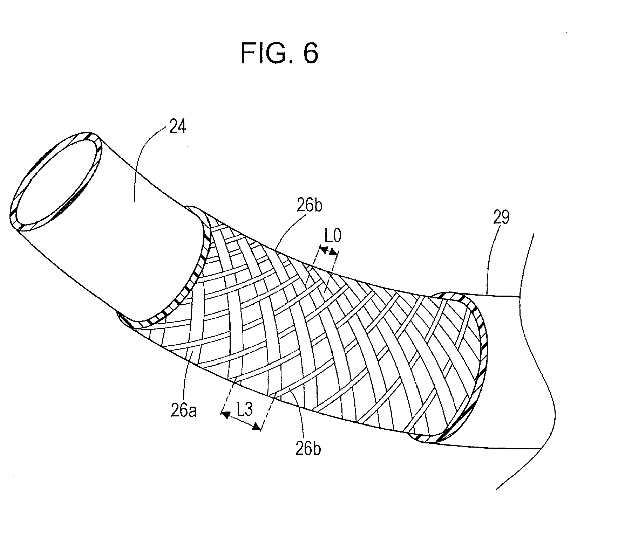

The braid 26, serving as a reinforcing member, is formed at an outer periphery of the inner layer 24. As shown in Figs. 2 and 6, the braid 26 is one in which first wires 26a and second wires 26b are braided in the form of meshes. In the embodiment, a total of 16 wires (eight first wires 26a and eight second wires 26b) are alternately braided. That is, the first wires 26a are wound in one direction, and the second wires 26b are wound in another direction.

The combination of first wires 26a and second wires 26b of the braid 26 is not limited to eight first wires 26a and eight second wires 26b. It may be a symmetrical combination such as four first wires 26a and four second wires 26b or two first wires 26a and two second wires 26b; or an asymmetrical combination such as four first wires 26a and eight second wires 26b or two first wires 26a and four second wires 26b. The width of the first wires 26a and the width of the second wires 26b may be the same, or the width of the first wires 26a may be greater than the width of the second wires 26b. Further, although the first wires 26a and the second wires 26b are alternately braided, they may be braided one by one.

The first wires 26a and the second wires 26b may be formed of the same material or different materials. Although, in the embodiment, the first wires 26a formed of stainless steel (SUS316) and having a low melting point and the second wires 26b formed of tungsten and having a high melting point are used, the materials of the first wires 26a and the second wires 26b are not particularly limited. For example, the first wires 26a and the second wires 26b may be formed of materials other that metal, such as reinforced plastic. However, in order to make it easier for, for example, a doctor to determine the position of the catheter 1 during examination or treatment, it is desirable that either of the first wires 26a or the second wires 26b be formed of a heavy metal (such as tungsten) material that is not transparent with respect to radiation.

The intermediate layer 29, formed of a resin, is formed at an outer periphery of the braid 26, and covers the inner layer 24 and the braid 26. The resin material of which the intermediate layer 29 is formed is not particularly limited, and is, for example, polyamide, polyamide elastomer, polyester, or polyurethane.

As shown in the cross sectional view of Fig. 3, a body 10a of the catheter shaft 10, a distal end portion 10b that is adjacent to the body 10a, and a tip portion 10c that is adjacent to the distal end portion 10 are covered with the intermediate layer 29 in such a manner as to exclude the distal-end tip 12. The intermediate layer 29 is bonded to the inner layer 24 at gaps in the braid 26 (in other words, regions where the first wires 26a and the second wires 26b do not overlap each other).

At an outer surface of the intermediate layer 29, protrusions 31 are formed at locations where the braid 26 exists and recesses 32 are formed at the gaps in the braid 26. Therefore, the intermediate layer 29 has an uneven contour.

The outer layer 28, formed of a resin, is formed at an outer periphery of the intermediate layer 29, and covers the intermediate layer 29. The resin material of which the outer layer 28 is formed is not particularly limited. Similarly to the intermediate layer 29, the resin material may be, for example, polyamide, polyamide elastomer, polyester, or polyurethane. The outer layer 28 is formed of resin materials having different hardnesses so as to be flexible from the body 10a to the tip portion 10c of the catheter shaft 10. Therefore, a first outer layer 28a that covers the body 10a is formed of a resin having a high rigidity, a second outer layer 28b that covers the distal-end portion 10b is formed of a resin that is more flexible than the first outer layer 28a, and a third outer layer 28c that covers the tip portion 10c is formed of a resin that is more flexible than the second outer layer 28b. The first outer layer 28a, the second outer layer 28b, and the third outer layer 28c are bonded to the intermediate layer 29 while they are disposed in the recesses 32 of the intermediate layer 29.

Although, in the sectional view of Fig. 3, the catheter shaft 10 has a shape having the same inside diameter in an axial direction, the shape of the catheter shaft 10 is not limited thereto. The catheter shaft 10 may have a shape in which only the tip portion 10c of the catheter shaft 10 is caused to have a small diameter by forming the catheter shaft 10 from the tip portion 10c towards the body 10a so as to taper with increasing diameter.

The distal-end tip 12, formed of a resin, is mounted to a distal end of the catheter shaft 10. The distal-end tip 12 is a cylindrical member including a distal-end opening 15. The resin of which the distal-end tip 12 is formed is not particularly limited, and is, for example, polyurethane or polyurethane elastomer. The distal-end tip 12 may contain heavy metal powder that is not transparent with respect to radiation. For example, when the distal-end tip 12 contains heavy metal powder (such as tungsten powder) that is not transparent with respect to radiation in a range of from approximately 65w% to approximately 90w%, it is possible for, for example, a doctor to determine the precise location of the catheter 1 during examination or treatment.

Next, the state in which the catheter 1 according to the embodiment is inserted into a meandering blood vessel and curved is explained.

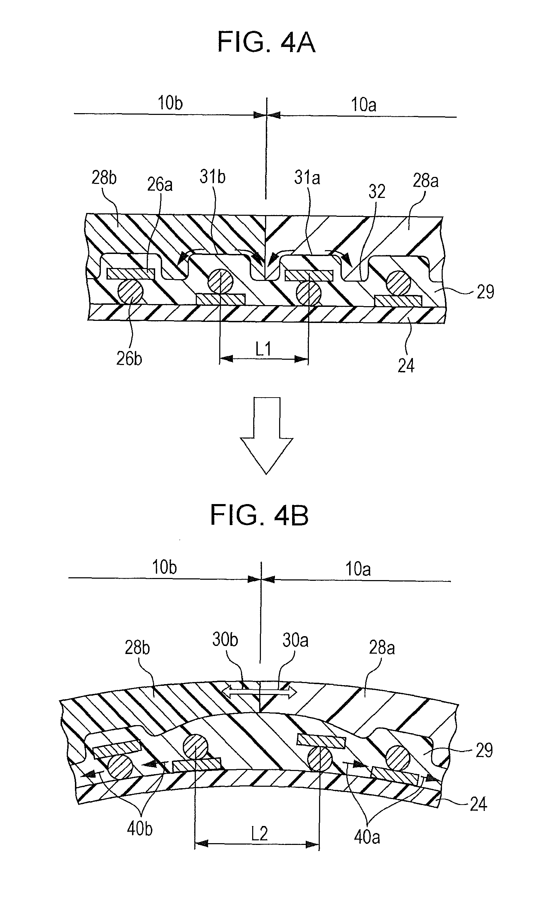

As shown in Fig. 4A, when the catheter 1 is not subjected to an external force and is not curved, the braid 26 is formed at an equal interval of pitch L1. When the catheter 1 is curved, a portion of the braid 26 that is positioned at the body 10a of the catheter shaft 10 tries to move in the direction of a proximal end (rightwards in Fig. 4B) and a portion of the braid 26 that is positioned at the distal end portion 10b of the catheter shaft 10 tries to move in the direction of a distal end (leftwards in Fig. 4B). Therefore, if the braid 26 is not restrained by the intermediate layer 29, the first outer layer 28a, and the second outer layer 28b, as shown in Fig. 6, the pitch interval at the outer side of the braid 26 increases from L1 to L3, while the pitch interval at the inner side of the braid 26 is decreased from L1 to L0. As a result, for example, by the movement of the braid 26 whose pitch interval is increased from L1 to L3, a tensile stress 40a towards the proximal end and a tensile stress 40b towards the distal end are generated at the intermediate layer 29 (see Fig. 4B).

In the catheter 1 according to the embodiment, the thickness of the intermediate layer 29 is constant from the body 10a to the tip portion 10c. Therefore, the adhesive strength (adhesiveness) between the intermediate layer 29 and the inner layer 24 is uniform from the body 10a to the tip portion of the catheter shaft 10. Therefore, the suppression of the movement of the portion of the braid 26 at the body 10a by the intermediate layer 29 and the suppression of the movement of the portion of the braid 26 at the distal end portion 10b by the intermediate layer 29 are equal to each other. The tensile stress 40a towards the proximal end and the tensile stress 40b towards the distal end are in equilibrium, and the increase in the pitch interval of the braid 26 is restricted so that the pitch interval increases to L2 instead of to L3. Therefore, it is possible to reduce the concentration of the stress at a boundary between the body 10a and the distal end portion 10b. The relationship between the pitch intervals is L0 < L1 < L2 < L3.

As shown in Fig. 4B, in the catheter 1 according to the embodiment, the intermediate layer 29 is formed of a resin whose rigidity is higher than that of the second outer layer 28b (in other words, the intermediate layer 29 is formed of a resin having a certain rigidity). Even if, when the catheter 1 is curved, the braid 26 is moved by the curving of the catheter 1, since the intermediate layer 29 has a certain rigidity, it is possible to suppress the movement of the braid 26 and cause the pitch interval to be restricted to L2. Since the intermediate layer 29 is provided with a certain rigidity, it is possible to adjust the movement of the braid 26 that cannot be restrained by the flexible second outer layer 28b.

With the movement of the braid 26, some of the protrusions 31 at the intermediate layer 29 move into the recesses 32 that are adjacent thereto. For example, if, among the protrusions 31 at the intermediate layer 29, protrusions existing near the boundary between the body 10a and the distal end portion 10b are protrusions 31a and 31b, respectively, when the catheter 1 is curved, the intermediate layer 29 at the protrusions 31a and 32b moves into the recess 32 between the protrusion 31a and the protrusion 31b while being stretched in an axial direction. Therefore, with the movement of the braid 26, the intermediate layer 29 can be stretched in the axial direction. This causes the intermediate layer 29 to reduce the tensile stresses 40a and 40b generated by the braid 26, thereby making it possible to reduce the concentration of stress that is generated at a boundary between the first outer layer 28a and the second outer layer 28b.

Even if, when the catheter 1 is curved, the tensile stress 30a towards the proximal end acts upon the first outer layer 28a at the body 10a and the tensile stress 30b towards the distal end acts upon the second outer layer 28b at the distal end portion 10b, since the first outer layer 28a and the second outer layer 28b are bonded to the recesses 32 in the intermediate layer 29, it is possible to make it unlikely for the first outer layer 28a and the second outer layer 28b to be peeled off from the intermediate layer 29 due to an anchor effect.

Accordingly, even if the catheter 1 is placed in a curved state for a long time, the intermediate layer 29 having a constant thickness from the body 10a to the distal end portion 10b and having an uneven contour makes it possible to suppress the movement of the braid 26 and to make it unlikely for the first outer layer 28a and the second outer layer 28b to be peeled off from the intermediate layer 29.

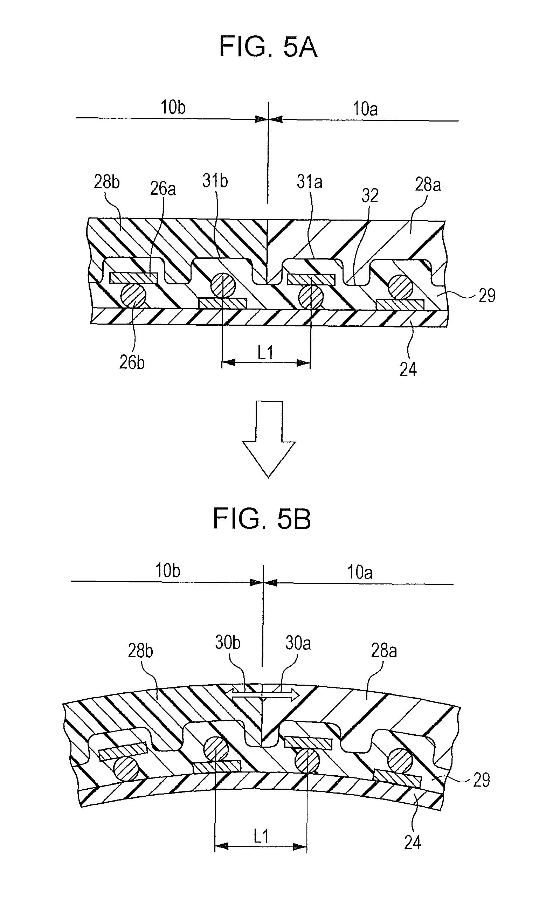

In the catheter 1 according to another embodiment, as shown in Figs. 5A and 5B, an intermediate layer 29 may be formed of a resin having a rigidity that is higher than those of a first outer layer 28a and a second outer layer 28b. The points in Figs. 5A and 5B differing from those in Figs. 4A and 4B are described. By using the intermediate layer 29 having a rigidity that is higher than those of the first outer layer 28a and the second outer layer 28b, when the catheter 1 is curved, it is possible to further suppress the movement of a braid 26 (for example, an increase in the pitch interval of the braid 26) caused by the curving of the catheter 1, and to prevent the braid 26 from generating tensile stresses 40a and 40b themselves. However, when a resin having a high rigidity is used in the intermediate layer 29, the rigidity of a catheter shaft 10 from a body 10a to a tip portion 10c is increased. Therefore, it is desirable to make the intermediate layer 29 as thin as possible.

As shown in Figs. 3 to 5B, the shape of each second wire 26b of the braid 26 is circular in cross section and the shape of each first wire 26a of the braid 26 is rectangular in cross section and is longer than the diameter of the circular cross section shape of each second wire 26b. When a high-tension metal is used as the material of each first wire 26a and each second wire 26b, the formed braid 26 itself tries to widen in a peripheral direction (a direction that is orthogonal to the axial direction). When the braid 26 is curved, a stress that tries to separate the intermediate layer 29 from the inner layer 24 is generated in the peripheral direction. Accordingly, by causing a portion of the intermediate layer 29 to be moved into a location between a rectangular first wire 26a and a circular second wire 26b, it is possible to reduce peeling of the intermediate layer 29 from the inner layer 24 due to an anchor effect even if a peripheral stress is generated when the catheter 1 is curved.

Although not shown, the first wires 26a and the second wires 26b of the braid 26 may both be rectangular in cross section. This makes it possible to increase friction resistance by increasing the contact area between the first wires 26a and the second wires 26b and to reduce the tensile stresses 40a and 40b generated by the braid 26 by suppressing the movement of the braid (in other words, the increase in the pitch interval of the braid 26) caused by the curving of the catheter 1.

As shown in Figs. 3 to 5B, the boundary between the body 10a and the distal end portion 10b (the boundary between the first outer layer 28a and the second outer layer 28b) is disposed in a corresponding one of the recesses 32 in the intermediate layer 29. By this, when the catheter 1 is curved, a proximal end of the second outer layer 28b (that is located near the boundary) is caught by the protrusion 31b of the intermediate layer 29, so that, by the anchor effect, it is possible to make it further unlikely for the second outer layer 28b to be peeled off from the intermediate layer 29. Due to the same reason, a boundary between the distal end portion 10b and the tip portion 10c (boundary between the second outer layer 28b and the third outer layer 28c) is disposed in the corresponding one of the recesses 32 in the intermediate layer 29.

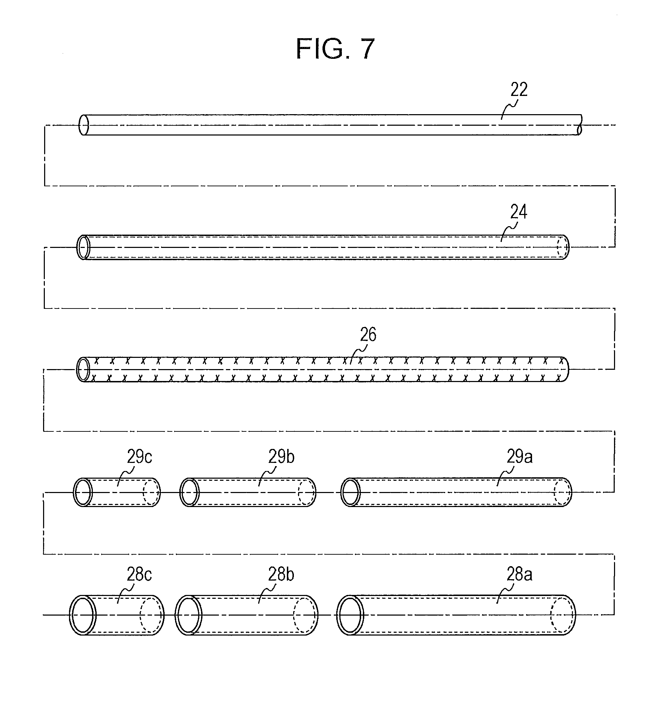

Next, the steps of forming the catheters 1 according to the embodiments are described with reference to Fig. 7.

First, in inner layer 24 is formed on a mandrel 22. Next, first wires 26a and second wires 26b are braided at an outer periphery of the inner layer 24, so that a braid 26 is formed. Then, an intermediate layer 29a and a first outer layer 28a having a high rigidity, an intermediate layer 29b having the same rigidity as the intermediate layer 29a and a second outer layer 28b that is more flexible than the first outer layer 28a, and an intermediate layer 29c having the same rigidity as the intermediate layer 29a and a second outer layer 28c that is even more flexible than the first outer layer 28b are successively inserted into an outer periphery of the braid 26. Then, the inner layer 24, the intermediate layer 29, and the outer layer 28 (the first outer layer 28a, the second outer layer 28b, and the third outer layer 28c) are welded by heating them to a predetermined temperature. At this time, the intermediate layers 29a, 29b, and 29c are formed of the same material. Therefore, it is possible to form the intermediate layer 29 with which the braid 26 is covered from a body 10a to a tip portion 10c of a catheter shaft 10.

A distal-end tip 12 is formed at the catheter shaft 10 by covering a distal end of the braid 26 with a tube that is formed of a resin and that is to become the distal-end tip 12, heating the tube to a predetermined temperature, and welding the tube to the distal end of the braid 2. Thereafter, by removing the mandrel 22, it is possible to form the catheter 1 including the catheter shaft 10 and the distal-end tip 12.

Although, in the description above, attention is focused on the boundary between the body 10a and the distal end portion 10b, which is an adjacent portion adjacent to the body 10a, of the catheter shaft 10, it is the same when the boundary is between the distal end portion 10b and the tip portion 10c, which is an adjacent portion adjacent to the distal end portion 10b, of the catheter shaft 10. In addition, it is the same even if a combination of the body 10a and the distal end portion 10b of the catheter shaft 10 is considered to be the body, and the tip portion 10c, which is an adjacent portion adjacent to the body, is considered to be the distal end portion. That is, the present invention is applicable to any portion as long as it is a boundary between outer layers having different hardnesses.

Although, in the description above, the braid 26 is covered with the intermediate layer 29 over the entire length from the proximal end to the distal end of the catheter shaft 10, the form thereof is not limited thereto. Any form may be used as long as the intermediate layer 29 having a constant thickness and having an uneven contour is provided near the boundary between outer layers having different hardnesses. Therefore, even if the distal end or the proximal end of the braid 26 is not covered with the intermediate layer 29, the advantageous effects of the present invention described above are obtained.

Although, in the description above, three different types of outer layers (the first outer layer 28a, the second outer layer 28b, and the third outer layer 28c) having different thicknesses are used for the catheter shaft 10, the number of types of outer layers is not particularly limited. The number of types of outer layers to be used may be changed as required.

As described above, in the catheters 1 according to the embodiments, since the thickness of the intermediate layer 29 is constant from the body 10a to the distal end portion 10b, the adhesive strength (adhesiveness) between the intermediate layer 29 and the inner layer 24 is uniform from the body 10a to the distal end portion 10b of the catheter shaft 10. Therefore, when the catheter 1 is curved, the movement of the braid 26 (in other words, an increase in the pitch interval of the braid 26) caused by the curving of the catheter can be uniformly suppressed from the body 10a to the distal end portion 10b. In addition, even if, when the catheter 1 is curved, the tensile stress 30a towards the proximal end acts upon the first outer layer 28a at the body 10a and the tensile stress 30b towards the distal end acts upon the second outer layer 28b at the distal end portion 10b, the first outer layer 28a and the second outer layer 28b are caught by the uneven contour of the intermediate layer 29 by an anchor effect, so that it is possible to prevent peeling of the first outer layer 28a and the second outer layer 28b from the intermediate layer 29.

## Claims

1. A catheter (1) comprising a catheter shaft (10) which includes:
an inner layer (24) formed from a body (10a) of the catheter shaft (10) to an adjacent portion (10b) of the catheter shaft (10), that is adjacent to the body (10a), the inner layer (24) being formed of a resin;
a braid (26) formed at an outer periphery of the inner layer (24) from the body (10a) to the adjacent portion (10b);
an intermediate layer (29) with which an outer periphery of the braid (26) is covered from the body (10a) to the adjacent portion (10b);
a first outer layer (28a) with which an outer periphery of the intermediate layer (29) is covered at the body (10a), the first outer layer (28a) being formed of a resin; and
a second outer layer (28b) with which an outer periphery of the intermediate layer (29) is covered at the adjacent portion (10b), the second outer layer (28b) being formed of a resin that is more flexible than the first outer layer (28a),
wherein the intermediate layer (29) has a contour alternately including protrusions (31) and recesses (32) from the body (10a) to the adjacent portion (10b), the protrusions (31) being formed at a radial outer side of the braid (26), the recesses (32) being each formed at a gap of the braid (26) adjacent to a corresponding one of the protrusions (31), and
wherein a radial thickness of the intermediate layer (29) at the gaps is constant from the body (10a) to the adjacent portion (10b).

2. The catheter according to Claim 1, wherein the intermediate layer (29) is formed of a resin having a rigidity that is higher than that of the second outer layer (28b).

3. The catheter according to Claim 2, wherein the rigidity of the resin forming the intermediate layer (29) is higher than those of the first outer layer (28a) and the second outer layer (28b).

4. The catheter according to any one of Claims 1 to 3, wherein the braid (26) is formed of a first wire (26a) and a second wire (26b), and a shape of the first wire (26a) and a shape of the second wire (26b) are both rectangular in cross section.

5. The catheter according to any one of Claims 1 to 3, wherein the braid (26) is formed of a first wire (26a) and a second wire (26b), a shape of one of the first wire (26a) and the second wire (26b) is circular in cross section, and a shape of the other of the first wire (26a) and the second wire (26b) is rectangular in cross section, the rectangular shape having a side that is longer than a diameter of the circular shape.

6. The catheter according to any one of Claims 1 to 5, wherein a boundary between the body (10a) and the adjacent portion (10b) is disposed at one of the recesses (32) of the intermediate layer (29).
